# FASCICULE DE BREVET EUROPEEN

(11) **EP 0 564 329 B1**
(45) Date de publication et mention de la délivrance du brevet: **03.12.1997**
(21) Numéro de dépôt: 93400774.1
(22) Date de dépôt: 24.03.1993
(51) Int. Cl.: B01J 23/62, B01J 23/825, B01J 37/025, C10G 45/40, C07C 7/163

(54) **Catalyseur contenant un métal du groupe VIII et un métal du groupe IIIA déposés sur un support**
Gruppe-VIII Metal und Gruppe-IIIA Metal an einem Träger enthaltender Katalysator
Catalyst containing group VIII metal and group IIIA metal on a support

(30) Priorité: 02.04.1992 FR 9204151
(43) Date de publication de la demande: 06.10.1993
(73) Titulaire: INSTITUT FRANCAIS DU PETROLE, 92502 Rueil-Malmaison (FR)
(72) Inventeur: Sarrazin, Patrick, F-92500 Rueil Malmaison (FR); Boitiaux, Jean-Paul, F-78300 Poissy (FR)

(56) Documents cités:
- WO-A-89/04818
- DE-B- 1 004 316
- FR-A- 2 091 114
- FR-A- 2 103 122
- FR-A- 2 594 711

## Description

La présente invention concerne un catalyseur renfermant un support, au moins un métal du GVIII et au moins un métal du GIIIA choisi parmi le gallium et l'indium, dans lequel le support est changé avec le(s) métal(aux) du GIIIA puis avec le(s) métal(aux) du GVIII.

Des catalyseurs a base de métaux du GVIII et du GIIIA sont déjà connus. Ainsi le brevet FR-A-2.103.122 décrit un tel catalyseur utilisable pour des réactions de déshydrogénation et de déshydrocyclisation. Selon ce brevet, l'ordre d'introduction de ces métaux est sans importance (page 3 lignes 13 à 19).

Dans le le brevet FR-A-2.091.114, des catalyseurs regénérables sont préparés, par incorporation d'au moins un métal dit supplémentaire catalytiquement inactif au support, calcination, introduction du métal du GVIII puis du métal supplémentaire catalytiquement actif (par exemple indium). Ce brevet enseigne que le métal du GVIII doit obligatoirement être introduit avant l'indium car sinon "l'influence avantageuse du métal supplémentaire sur le comportement du catalyseur ne se manifeste pas". De tels catalyseurs sont appliqués à la déshydrocyclisation et au réformage.

La demande de brevet WO-89/04818 décrit une composition catalytique comprenant un métal hydrogénant, et un matériau cristallin microporeux non-acide (tel que aluminosilicate comme ZSM-5, ALPO, argiles, silicates et titanates en feuillets) dans lequel se trouve un métal additionnel (In, Sn, Tl, ou Pb).

Il a été maintenant découvert que, contrairement à l'enseignement de l'art antérieur, des catalyseurs préparés selon un ordre d'introduction des éléments déterminés (GVIII puis GIIIA) non divulgués préalablement montrent des propriétés (activité, sélectivité) supérieures aux catalyseurs préparés soit par introduction simultanée des éléments des GIIIA et GVIII soit par introduction du GIIIA puis du GVIII.

Ces propriétés améliorées sont mises en évidence en hydrogénation sélective.

De nombreux procédés de production d'oléfines, tels que le vapocraquage, le craquage catalytique et la viscoréduction produisent des coupes polluées par des molécules plus insaturées que les oléfines recherchées. La bonne utilisation de ces coupes pour la fabrication de produits finis implique l'élimination de ces molécules qui contiennent des doubles liaisons conjuguées et/ou des triples liaisons. L'hydrogénation sélective de celles ci en oléfines correspondantes est le procédé de choix pour s'en débarrasser tout en récupérant les oléfines recherchées.

Ces réactions d'hydrogénation sont généralement effectuées dans une gamme de températures comprises entre 20 et 200°C, sous une pression comprise entre 10 et 100 bar (1 et 10 Mégapascal) et avec une vitesse spatiale horaire comprise entre 1 et 40 m³/m³ de catalyseur/h. Les catalyseurs généralement utilisés sont constitués d'un ou plusieurs métaux déposés sur un support oxyde. Les métaux de base préférés sont couramment ceux du groupe VIII et plus particulièrement le nickel, le palladium et le platine. Les supports sont, quant à eux, souvent choisis parmi l'alumine, la silice, les silice-alumines, les aluminates ou encore le charbon.

La mise en oeuvre industrielle de tels catalyseurs se fait souvent en présence d'additifs visant à l'amélioration de la sélectivité de la réaction d'hydrogénation. Le composé le plus fréquemment employé étant le monoxyde de carbone comme revendiqué dans le brevet EP 0.081.041.

Le développement de catalyseurs plus performants du point de vue de l'activité et de la sélectivité a amené l'introduction d'autres métaux dans les formulations catalytiques. On peut citer, par exemple, l'argent (US-A-4409410 de la demanderesse) et l'or (US-A-4490481 de la demanderesse) qui améliorent très nettement les propriétés catalytiques des métaux du groupe VIII pour la réaction d'hydrogénation.

Il est montré dans la présente invention qu'il est possible de réaliser l'hydrogénation de composés insaturés dioléfiniques et acétyléniques avec des sélectivités élevées en composés oléfiniques correspondants sans diminution de l'activité du métal de base (c'est-à-dire du groupe VIII), avec promotion et ceci sans additif dans le milieu réactionnel ni préparation d'un alliage bimétallique. On opère dans un réacteur continu ou discontinu en présence d'hydrogène sous une pression totale comprise entre 10 et 100 bars (1 et 10 Mégapascal) et de préférence entre 20 et 80 bar (2 et 8 Mégapascal), bien que l'on puisse opérer sans inconvénient, par exemple, jusqu'à 300 bar (30 Mégapascals), à une température comprise entre 0 et 200 degrés Celsius et de préférence entre 30 et 120 degrés Celsius en présence d'un nouveau catalyseur métallique. Ledit catalyseur renferme (a) au moins un métal du groupe VIII choisi parmi le nickel, le palladium, le platine, le rhodium et le ruthénium (le palladium, le platine et le nickel étant les métaux préférés) et dont le pourcentage pondéral est choisi entre 0,1 et 10 % et de préférence entre 0,2 et 5 % et (b) au moins un élément additionnel métallique choisi dans le groupe III.A constitué par le gallium et l'indium dont le pourcentage pondéral est choisi entre 0,01 et 10 % et de préférence entre 0,1 et 5 % et le rapport molaire élément métallique du groupe III sur métal du groupe VIII est avantageusement compris entre 0,2 et 5 et de préférence entre 0,3 et 2 (c) un support, choisi dans le groupe constitué par une silice, une alumine, une silice-alumine, un charbon, et les aluminates des éléments des groupes I.A, II.A ou II.B de la classification périodique comme par exemple les aluminates de Ca, Mg, Ba, Zn, Na, K, Cd et les aluminates mixtes des éléments alcalins, alcalino-terreux, du zinc ou du cadmium.

Le catalyseur peut être préparé par différentes procédures. Une procédure préférée est l'imprégnation du support, mais l'invention n'est pas limitée à une procédure déterminée.

L'imprégnation consiste, par exemple, à mettre en contact le support préformé et une solution aqueuse ou organique d'un composé du métal ou des métaux choisi(s) dans le groupe III.A (gallium et l'indium) le volume de solution étant en excès par rapport au volume de rétention du support ou de préférence égal à ce volume. Après avoir laissé le contact entre le support et la solution pendant plusieurs heures, le support imprégné est filtré, lavé à l'eau distillé, séché et calciné sous air habituellement entre 110°C et 600°C et de préférence entre 110°C et 500°C. Avant dépôt du métal ou des métaux du groupe VIII, on peut avantageusement réduire le catalyseur sous hydrogène, on opère habituellement entre 50°C et 600°C et de préférence entre 90°C et 500°C ou à l'aide d'un réducteur organique en solution. Cette opération permet encore d'augmenter l'activité du catalyseur.

Le produit obtenu est alors imprégné par une solution organique (hydrocarbonée par exemple) ou aqueuse d'un métal du groupe VIII selon la nature du précurseur utilisé ; d'une manière particulièrement avantageuse, on utilise une solution de nitrate de palladium ou de nickel dans l'eau.

Le support ainsi imprégné est filtré, éventuellement lavé à l'eau distillée puis séché et calciné sous air habituellement entre environ 110°C et environ 600°C, et de préférence entre environ 110°C et environ 500°C, puis ensuite réduit sous hydrogène à une température habituellement comprise entre environ 50°C et environ 600°C et de préférence entre environ 80°C et environ 500°C. Les éléments des GVIII et GIII se trouvent alors sous forme d'oxyde et/ou métallique déposés sur le support.

Une autre méthode consiste à malaxer la poudre humide de support avec les précurseurs du catalyseur et à mettre ensuite en forme et sécher.

Les exemples des précurseurs métalliques utilisables dans la préparation du catalyseur sont les suivants:

Pour le métal du groupe VIII, on peut utiliser des composés tels que les chlorures, les nitrates, les composés halogèno-aminés, les composés aminés, les sels d'acides organiques solubles dans le solvant d'imprégnation.

On peut aussi utiliser des composés organométalliques d'un métal du groupe VIII en solution dans un solvant organique, par exemple un hydrocarbure. Comme exemple d'hydrocarbures on peut citer les hydrocarbures paraffiniques saturés dont la chaîne hydrocarbonée renferme de 6 à 12 atomes de carbone par molécule, les hydrocarbures naphténiques qui renferment de 6 à 12 atomes de carbone par molécule ou encore les hydrocarbures aromatiques renfermant de 6 à 12 atomes de carbone par molécule. A titre d'exemples de composés organométalliques de métal du groupe VIII on peut citer : les composés carbonyles, halogénocarbonyles et les acétylacétonates sans que cette liste soit limitative.

L'élément choisi dans le groupe constitué par le gallium et l'indium peut être introduit de préférence sous la forme d'au moins un composé inorganique choisi dans le groupe formé par les chlorures, les nitrates, les composés halogéno-aminés, les composés aminés et les sels d'acides organiques solubles dans les solvants d'imprégnation.

L'introduction du métal III.A est avantageusement effectuée à l'aide d'une solution aqueuse du composé inorganique du dit métal III.A.

L'élément choisi dans le groupe constitué par le gallium et l'indium peut aussi être introduit par l'intermédiaire de composés organométalliques en solution dans un solvant organique, par exemple un hydrocarbure. Comme exemple d'hydrocarbures on peut citer les hydrocarbures paraffiniques saturés dont la chaîne hydrocarbonée renferme de 6 à 12 atomes de carbone par molécule, les hydrocarbures naphténiques qui renferment de 6 à 12 atomes de carbone par molécule ou encore les hydrocarbures aromatiques renfermant de 6 à 12 atomes de carbone par molécule. A titre d'exemples de composés organométalliques de métal du groupe constitué par le gallium et l'indium on peut citer : les alkyles, les alkoxydes, les acétates et les acétylacétonates sans que cette liste soit limitative.

Le support peut être de nature variée, comme déjà mentionné plus haut. Un support particulièrement adapté possède des caractéristiques spécifiques telles qu'une aire spécifique, déterminée par la méthode B.E.T., comprise entre 10 et 500 m² par gramme et de préférence comprise entre 50 et 500 m² par gramme et un volume poreux total de 0,2 à 1,3 cm³ par gramme de support.

Une fois les métaux fixés sur le support, le catalyseur subit avantageusement un traitement d'activation sous hydrogène à haute température, par exemple 50-600°C, afin d'obtenir une phase métallique active. La procédure de ce traitement sous hydrogène consiste par exemple en une montée lente de la température sous courant d'hydrogène jusqu'à la température maximale de réduction, comprise par exemple entre 50°C et 600°C et de préférence entre 80°C et 500°C, suivie d'un maintien pendant par exemple 1 à 6 heures à cette température.

Les exemples suivants, non limitatifs, illustrent l'invention.

### EXEMPLE 1 (comparatif)

On se propose dans cet exemple d'hydrogéner une charge constituée de 10 % poids de butadiène dans de l'heptane. La réaction est mise en oeuvre dans un réacteur discontinu parfaitement agité de type Grignard dans les conditions opératoires suivantes :
Pression = 20 bar
Température = 20°C

L'hydrogène utilisé est exempt de monoxyde de carbone.

Le catalyseur utilisé, appelé catalyseur A, est constitué de palladium à une teneur de 0,3 % poids déposé sur une alumine de transition de surface spécifique égale à 70 m²/g. Il est préparé par imprégnation à sec d'une alumine gamma tétragonale de volume poreux égal à 0,6 cm³/g à l'aide d'une solution de nitrate de palladium. Après imprégnation, l'échantillon est séché à une température de 120°C pendant 2 heures, puis calciné sous débit d'air à une température de 450°C pendant 2 heures. Avant test, on réduit le catalyseur sous débit d'hydrogène à une température de 150°C pendant 2 heures.

Au cours de l'avancement de la réaction des échantillons sont régulièrement prélevés et analysés par chromatographie en phase gazeuse de manière à suivre la transformation du butadiène en butènes et butane. Les résultats obtenus sont présentés dans le tableau suivant :

| **Temps (minutes)** | **Butadiène (% poids)** | **Butènes (%poids)** | **Butane (%poids)** |
|---|---|---|---|
| 0 | 100 | - | - |
| 1 | 80,85 | 19,1 | 0,05 |
| 2 | 75,75 | 24,15 | 0,1 |
| 3 | 66 | 33,85 | 0,15 |
| 4 | 54,5 | 45,33 | 0,17 |
| 5 | 43 | 56,8 | 0,2 |
| 6 | 32 | 67,7 | 0,21 |
| 7 | 20,5 | 79,28 | 0,22 |
| 8 | 9 | 90,7 | 0,3 |

### EXEMPLE 2 (selon l'invention)

Dans cet exemple on met en oeuvre la même réaction dans les mêmes conditions que dans l'exemple 1, mais cette fois on utilise successivement différents catalyseurs contenant 0,3 % poids de palladium et une teneur variable en gallium. Le support utilisé est identique à celui du catalyseur A monométallique de l'exemple 1. Différents lots de ce support sont imprégnés à sec par des solutions de nitrate de gallium de concentrations variables. Après imprégnation, les échantillons sont séchés à une température de 120°C pendant 2 heures, puis calcinés sous débit d'air à une température de 450°C pendant 2 heures. On procède alors au dépôt du palladium en utilisant la même méthode que celle décrite dans l'exemple 1 pour le catalyseur A. Avant test, on réduit les catalyseurs sous débit d'hydrogène à une température de 150°C pendant 2 heures.

Le tableau suivant présente la composition du produit après 8 minutes de réaction pour chacun des catalyseurs repérés par leur teneur en gallium ainsi que pour le catalyseur A monométallique de l'exemple 1.

| **Teneur en Ga (% poids)** | **Butadiène (% poids)** | **Butènes (%poids)** | **Butane (%poids)** |
|---|---|---|---|
| 0 | 9 | 90,7 | 0,3 |
| 0,07 | 6,57 | 93,16 | 0,27 |
| 0,21 | 5,55 | 94,2 | 0,25 |
| 0,42 | 7,5 | 92,22 | 0,28 |
| 0,82 | 8 | 91,7 | 0,3 |
| 1,19 | 10,3 | 89,4 | 0,3 |

On remarque que les échantillons ayant une teneur en gallium comprise entre 0,07 et 0,82 % poids présentent une activité supérieure à celle du catalyseur monométallique puisque au bout du même temps de réaction (8 minutes) la teneur en butadiène du produit est inférieure. De plus, on note que ces catalyseurs plus actifs que le monométallique sont aussi plus sélectifs vis à vis de l'hydrogénation consécutive des butènes. En effet, bien que la conversion du butadiène soit plus importante, on voit que la teneur en butènes est plus importante et que la formation de butane est diminuée.

### EXEMPLE 3 (selon l'invention)

Dans cet exemple on met en oeuvre la même réaction dans les mêmes conditions que dans l'exemple 1. On utilise cette fois un catalyseur B contenant 0,3 % poids de palladium et 0,24 % poids de gallium déposés sur le même support que dans l'exemple 1 suivant la même méthode de préparation que dans l'exemple 2. On utilise aussi un catalyseur C ayant la même composition mais qui diffère du catalyseur B par le fait que le précurseur au gallium sur alumine utilisé lors de la préparation a été réduit sous hydrogène à une température de 450°C pendant 2 heures avant le dépôt de palladium. La composition des produits obtenus après 8 minutes de réaction est présentée dans le tableau suivant :

| **Catalyseur** | **Butadiène (% poids)** | **Butènes (%poids)** | **Butane (%poids)** |
|---|---|---|---|
| **B** | 5,6 | 94,14 | 0,26 |
| **C** | 4,2 | 95,53 | 0,27 |

On voit que le catalyseur C est plus actif que le catalyseur B vis à vis de l'hydrogénation du butadiène. Un gain de sélectivité est aussi observé puisqu'avec une conversion en butadiène plus importante, la teneur en butènes est plus élevée pour le catalyseur C.

### EXEMPLE 4 (comparatif)

On se propose dans cette exemple d'hydrogéner une coupe C₃ de vapocraquage dont la composition est la suivante :
Propane = 3,59 %
Propylène = 92,14 %
Propyne (MA) = 1,78 %
Propadiène (PD) = 1,65 %

La réaction est mise en oeuvre en phase liquide dans un réacteur continu à lit fixe dans les conditions opératoires suivantes :
Pression = 24 bar
Température = 50°C
Vitesse spatiale horaire = 20 cm³ de charge/cm³ de catalyseur/h
Rapport molaire hydrogène sur propyne plus propadiène = 1,2

L'hydrogène utilisé est exempt de monoxyde de carbone. Le catalyseur utilisé est le catalyseur A de l'exemple 1.

Au cours du temps des échantillons de produit sont régulièrement prélevés et analysés par chromatographie en phase gazeuse de manière à suivre la conversion du propyne et du propadiène ainsi que la teneur en propylène. Les résultats obtenus sont présentés dans le tableau suivant :

| **Temps (heures)** | **Propylène (%poids)** | **Propyne (%poids)** | **Propadiène (%poids)** |
|---|---|---|---|
| 50 | 94,87 | - | 0,071 |
| 100 | 94,92 | - | 0,069 |
| 150 | 95,13 | - | 0,080 |
| 200 | 94,78 | - | 0,052 |
| 300 | 94,90 | - | 0,065 |

Si l'on calcule les teneurs moyennes en propadiène et en propylène pendant l'opération, on peut calculer la conversion moyenne en propyne et en propadiène ainsi que le gain moyen en propylène. On trouve dans cet exemple une conversion de 98 % et un rendement propylène exprimé par le rapport de la teneur en propylène du produit sur la teneur en propylène de la charge de 103 %.

### EXEMPLE 5 (selon l'invention)

Dans cet exemple on met en oeuvre la même réaction dans les mêmes conditions que dans l'exemple 4, mais en présence du catalyseur C de l'exemple 3. Les résultats d'analyse obtenus sont présentés dans le tableau suivant :

| **Temps (heures)** | **Propylène (%poids)** | **Propyne (%poids)** | **Propadiène (%poids)** |
|---|---|---|---|
| 50 | 95,41 | - | 0,0072 |
| 100 | 95,43 | - | 0,0089 |
| 150 | 95,39 | - | 0,0070 |
| 200 | 95,05 | - | 0,0090 |
| 300 | 95,56 | - | 0,0060 |

On trouve dans cet exemple une conversion de 99,78 % et un rendement propylène exprimé par le rapport de la teneur en propylène du produit sur la teneur en propylène de la charge de 103,5 %

### EXEMPLE 6 (comparatif)

Dans cet exemple on met en oeuvre la même réaction dans les mêmes conditions que dans l'exemple 1, mais cette fois on utilise successivement différents catalyseurs contenant 0,3% poids de palladium et une teneur variable en gallium. Le support utilisé est identique à celui du catalyseur A monométallique de l'exemple 1. Différents lots de ce support sont imprégnés à sec par du nitrate de palladium en utilisant la même méthode que celle décrite dans l'exemple 1 pour le catalyseur A. On dépose ensuite le gallium par imprégnation de solutions de nitrate de gallium de concentrations variables. Après imprégnation, les échantillons sont séchés à une température de 120°C pendant 2 heures, puis calcinés sous débit d'air à une température de 450°C pendant 2 heures. Avant test, on réduit les catalyseurs sous débit d'hydrogène à une température de 150°C pendant 2 heures.

Le tableau suivant présente la composition du produit après 8 minutes de réaction pour chacun des catalyseurs repérés par leur teneur en gallium ainsi que pour le catalyseur A monométallique de l'exemple 1.

| **Teneur en Ga (% poids)** | **Butadiène (% poids)** | **Butènes (%poids)** | **Butane (%poids)** |
|---|---|---|---|
| 0 | 9 | 90,7 | 0,3 |
| 0,08 | 7,57 | 92,14 | 0,29 |
| 0,23 | 5,95 | 93,78 | 0,27 |
| 0,41 | 8,5 | 91,22 | 0,28 |
| 0,80 | 8,6 | 91,1 | 0,3 |
| 1,22 | 10,2 | 89,49 | 0,31 |

On remarque que les échantillons ayant une teneur en gallium comprise entre 0,08 et 0,80 % poids présentent une activité supérieure à celle du catalyseur monométallique de l'exemple 1 puisque au bout du même temps de réaction (8 minutes) la teneur en butadiène du produit est inférieure. De plus, on note que ces catalyseurs plus actifs que le monométallique sont aussi plus sélectifs vis à vis de l'hydrogénation consécutive des butènes. En effet, bien que la conversion du butadiène soit plus importante, on voit que la teneur en butènes est plus élevée et que la formation de butane est diminuée.

## Revendications

1. Catalyseur comprenant au moins un métal du groupe VIII et au moins un métal du groupe III-A choisi parmi le gallium et l'indium, lesdits métaux étant déposés sur le support de catalyseur, ledit support étant choisi dans le groupe formé par la silice, l'alumine, la silice alumine, les aluminates des éléments du groupe IA, IIA, IIB, les aluminates mixtes des éléments alcalins, alcalino-terreux, du zinc ou du cadmium, le charbon, ledit catalyseur étant caractérisé en ce qu'il est préparé par :
a) imprégnation du support avec une solution d'un composé du groupe III-A, la concentration en métal du groupe III-A étant choisie de façon à ce que la quantité de métal du groupe III-A fixée soit comprise entre 0,01 et 10 % en poids,
b) puis imprégnation du produit obtenu avec une solution d'un composé du groupe VIII, la concentration en métal étant choisie de façon à ce que la quantité de métal du groupe VIII fixée soit comprise entre 0,01 et 10 % en poids,
c) calcination du produit obtenu entre 110 et 600°C.

2. Catalyseur selon la revendication 1, caractérisé en ce que préalablement à l'étape (b), le produit est soumis à un traitement thermique en milieu oxydant entre 110 et 600°C.

3. Catalyseur selon la revendication 2, caractérisé en ce que le produit obtenu après traitement thermique en milieu oxydant est soumis à une réduction, préalablement à l'étape (b).

4. Catalyseur selon l'une des revendications précédentes, dans lequel le métal du groupe VIII est choisi dans le groupe constitué par le palladium, le platine et le nickel.

5. Catalyseur selon l'une des revendications précédentes, dans lequel la concentration en métal du groupe VIII est comprise entre 0,2 et 5 % en poids.

6. Catalyseur selon l'une des revendications précédentes, dans lequel la concentration en métal du groupe III-A est comprise entre 0,1 et 5 % en poids.

7. Catalyseur selon l'une des revendications précédentes, dans lequel le rapport molaire élément métallique additionnel du groupe III-A sur métal du groupe VIII est compris entre 0,2 et 5.

8. Catalyseur selon la revendication 7, dans lequel le rapport molaire est compris entre 0,3 et 2.

9. Utilisation du catalyseur, selon l'une des revendications précédentes, pour la conversion de charges hydrocarbonées.

10. Utilisation selon la revendication 9 pour l'hydrogénation sélective de charges contenant des composés insaturés dioléfiniques et/ou acétyléniques.

## Claims

1. Catalyst incorporating at least one group VIII metal and at least one group IIIA metal chosen from among gallium and indium, said metals being deposited on the catalyst support, said support being chosen from the group formed by silica, alumina,silica-alumina, aluminates of group IA, IIA, IIB elements, mixed alkali metal, alkaline earth, zinc or cadmium aluminates, charcoal, said catalyst being characterized in that it is prepared by :
a) impregnation of the support with a solution of a group IIIA compound, the group IIIA metal concentration being chosen in such a way that the group IIIA metal quantity fixed is between 0.01 and 10% by weight,
b) then impregnation of the product obtained with a solution of a group VIII compound, the metal concentration being chosen in such a way that the quantity of group VIII metal fixed is between 0.01 and 10% by weight,
c) calcination of the product obtained at between 110 and 600°C.

2. Catalyst according to claim 1, characterized in that prior to stage (b), the product undergoes a heat treatment in an oxidizing medium at between 110 and 600°C.

3. Catalyst according to claim 2, characterized in that the product obtained after heat treatment in an oxidizing medium undergoes a reduction, prior to stage (b).

4. Catalyst according to any one of the preceding claims, wherein the group VIII metal is chosen from among palladium, platinum and nickel.

5. Catalyst according to any one of the preceding claims, wherein the group VIII metal concentration is between 0.2 and 5% by weight.

6. Catalyst according to any one or the preceding claims, wherein the group IIIA metal concentration is between 0.1 and 5% by weight.

7. Catalyst according to any one of the preceding claims, wherein the molar ratio of the additional metallic element of group IIIA on the metal of group VIII is between 0.2 and 5.

8. Catalyst according to claim 8, wherein the molar ratio is between 0.3 and 2.

9. Use of the catalyst according to any one of the preceding claim for the conversion of hydrocarbon charges.

10. Use according to claim 10 for the selective hydrogenation of charges containing unsaturated diolefin and/or acetylene compounds.

## Patentansprüche

1. Katalysator, wenigstens ein Metall der Gruppe VIII und wenigstens ein Metall der Gruppe III-A umfassend, das gewählt ist aus Gallium und Indium, wobei die Metalle auf dem Katalysatorträger abgeschieden sind und der Träger gewählt ist aus der Gruppe, gebildet durch Siliciumoxid, Aluminiumoxid, Silicium(di)Oxid/Aluminiumoxid, die Aluminate der Gruppe IA, IIA, IIB, die gemischten Aluminate der Alkalielemente, der Erdalkalielemente, des Zinks oder des Cadmiums, die Kohle, wobei der Katalysator sich dadurch auszeichnet, daß er hergestellt ist durch
a) Imprägnieren des Trägers mit einer Lösung einer Zusammensetzung der Gruppe IIIA, wobei die Konzentration an Metall der Gruppe IIIA derart gewählt ist, daß die fixierte Metallmenge der Gruppe IIIA zwischen 0,01 und 10 Gew.-% beträgt,
b) dann Imprägnieren des erhaltenen Produkts mit einer Lösung einer Zusammensetzung der Gruppe VIII, wobei die Metallkonzentration derart gewählt ist, daß die fixierte Metallmenge der Gruppe VIII zwischen 0,01 und 10 Gew.-% beträgt, und
c) Calcinieren des erhaltenen Produktes zwischen 110 und 600° C.

2. Katalysator nach Anspruch 1, dadurch gekennzeichnet, daß vor der Stufe( b) das Produkt einer thermischen Behandlung in oxydierender Umgebung zwischen 110 und 600° C ausgesetzt ist.

3. Katalysator nach Anspruch 2, dadurch gekennzeichnet, daß das nach der thermischen Behandlung in oxydierender Umgebung erhaltene Produkt einer Reduktion vor der Stufe (b) ausgesetzt

4. Katalysator nach einem der vorhergehenden Ansprüche, bei dem das Metall der Gruppe VIII gewählt ist aus der Gruppe die durch Palladium, Platin und Nickel gebildet ist.

5. Katalysator nach einem der vorhergehenden Ansprüche, bei dem die Metallkonzentration der Gruppe VIII zwischen 0,2 und 5 Gew.-% beträgt.

6. Katalysator nach einem der vorhergehenden Ansprüche, bei dem die Metallkonzentration der Gruppe IIIA zwischen 0,1 und 5 Gew.-% beträgt.

7. Katalysator nach einem der vorhergehenden Ansprüche, bei dem das Mol-Verhältnis metallisches Zusatzelement der Gruppe IIIA zu Metall der Gruppe VIII zwischen 0,2 und 5 beträgt.

8. Katalysator nach Anspruch 7, bei dem das Mol-Verhältnis zwischen 0,3 und 2 beträgt.

9. Verwendung des Katalysators nach einem der vorhergehenden Ansprüche zur Umwandlung kohlenwasserstoffhaltiger Chargen.

10. Verwendung nach Anspruch 9 zur selektiven Hydrierung von ungesättigte diolefinische und/oder acetylenische Zusammensetzungen enthaltenden Chargen.
